# EUROPEAN PATENT SPECIFICATION

(11) **EP 3 653 186 B1**
(45) Date of publication and mention of the grant of the patent: **22.03.2023**
(21) Application number: 18832781.1
(22) Date of filing: 12.07.2018
(51) Int. Cl.: A61F 13/533, A61F 13/15, A61F 13/47, A61F 13/534

(54) **ABSORBENT ARTICLE**
ABSORBIERENDER ARTIKEL
ARTICLE ABSORBANT

(30) Priority: 14.07.2017 JP 2017137785
(43) Date of publication of application: 20.05.2020
(73) Proprietor: Daio Paper Corporation, Ehime 799-0492 (JP)
(72) Inventor: KURAMOCHI, Mihoko, Sakura-shi Tochigi 329-1411 (JP)
(74) Representative: Schön, Christoph
(86) International application number: PCT/JP2018/026350
(87) International publication number: WO 2019/013288

(56) References cited:
- EP-A1- 2 612 636
- WO-A1-2012/105533
- WO-A1-2015/076239
- WO-A1-2017/115673
- JP-A- H10 328 233
- JP-A- 2006 271 898
- JP-A- 2010 022 539
- JP-A- 2010 022 539
- JP-A- 2015 097 715
- JP-A- 2016 052 398
- JP-A- 2017 086 599

## Description

### Field

The present invention relates to an absorbent article such as a sanitary napkin, a panty liner, and an incontinence pad, and more particularly to an absorbent article in which a skin-side surface of a back side part of an absorbent body has a front-surface side concave groove recessed to the non-skin side and a plurality of partitioned absorption parts partitioned by the front-surface side concave groove and a non-skin-side surface of the back side part of the absorbent body has a gluteal cleft-facing concave groove recessed to the skin side.

### Background

Conventionally, as the absorbent article, there is known an absorbent article in which an absorbent body made of cotton-like pulp or the like is interposed between a liquid impermeable back-surface sheet such as a polyethylene sheet or a polyethylene sheet laminated nonwoven fabric and a liquid permeable front-surface sheet such as a nonwoven fabric or a permeable plastic sheet.

As the sanitary napkin for night use preventing leakage during sleep and the like, there are provided in the market many sanitary napkins including a thick absorbent body to secure an absorption amount. However, the thick absorbent body causes stiffening, whereby deteriorating wearing feeling. Recently, there have been demanded thinner sanitary napkins even for night use to improve wearing feeling. However, only if the absorbent body is simply made thinner, it is possible to cause leakage due to an insufficient absorbent amount and creeping leakage due to low fittedness on a skin surface, which has given uneasy feeling to a wearer.

Such a kind of absorbent article has been improved many times, and there have been developed absorbent articles capable of preventing leakage by improving the flexibility of the absorbent body and reducing a gap with a skin surface. For example, the following Patent Literature 1 discloses the absorbent article in which the absorbent core has a front-side middle-height region in the middle part in the width direction of the discharging area-facing section and a back-side middle-height region in the middle part in the width direction of the back side part. Each of the front-side middle-height region and the back-side middle-height region is divided by grooves that extend in the width direction of the absorbent core, and a plurality of small absorbent parts are formed along the longitudinal direction of the absorbent core.

Moreover, the following Patent Literature 2 discloses the absorbent article in which the absorption layer includes separation parts each having a groove on the skin-contact surface side, and a small absorption part partitioned by the separation parts and having a basis weight higher than that of the separation parts. The small absorption part includes, on the skin-noncontact-side surface, compressed concave parts toward the skin-contact surface side.

### Citation List

### Patent Literature

Patent Literature 1: Japanese Patent Application Laid-open No. 2015-97715
Patent Literature 2: Japanese Patent Application Laid-open No. 2014-97241

### Summary

### Technical Problem

However, in the above-described absorbent articles of Patent Literatures 1 and 2, each of the small absorption parts partitioned by the concave groove has a rectangular shape long in the longitudinal direction of the absorbent body. Therefore, the absorbent articles are hardly deformed along different curves of body parts, which has impeded sufficiently satisfiable fittedness.

Especially the sanitary napkin for night use is formed to be longer on the back side than that for normal use, so as to widely cover the gluteal of a wearer. Thus, it is required that the back side part of the absorbent article fits along the curve of the gluteal bulged outward and is easily deformed along the gluteal cleft in a recessed groove form.

Thus, the main object of the present invention is to provide an absorbent article capable of allowing the back side part of the absorbent body to be easily deformed along the shape of a body and improving fittedness.

### Solution to Problem

To solve the above-described problem, the invention according to claim 1 is an absorbent body, including at least an absorbent body, in which a skin side surface of a back side part of the absorbent body includes a front-surface side concave groove recessed to a non-skin side formed in a given pattern and a plurality of partitioned absorption parts partitioned by the front-surface side concave groove, and a non-skin-side surface of the back side part of the absorbent body includes a gluteal cleft-facing concave groove recessed to the skin side at a position corresponding to a gluteal cleft, the front-surface side concave groove includes a plurality of longitudinal direction concave grooves arranged along a substantially longitudinal direction of the absorbent article with intervals in the width direction and a plurality of width direction concave grooves arranged along a substantially width direction of the absorbent article with intervals in the longitudinal direction, and the longitudinal direction concave grooves are formed in a radial form inclined outward in the width direction from a front side toward a back side of the absorbent article.

In the above-described invention according to claim 1, the skin-side surface of the back side part of the absorbent body has the front-surface side concave groove recessed to the non-skin side in a given pattern and a plurality of partitioned absorption parts partitioned by the front-surface side concave groove. Thus, a part between the adjacent partitioned absorption parts is deformed to be bent inward, with the front-surface side concave groove as an origin. This allows the back side part of the absorbent body to be easily deformed flexibly along the curve of the gluteal bulged outward, thereby improving fittedness on the gluteal.

Moreover, the non-skin-side surface of the back side part of the absorbent body has the gluteal cleft-facing concave groove at a position corresponding to the gluteal cleft. Thus, with the gluteal cleft-facing concave groove as a flexible axis, the absorbent body is easily deformed in a convex shape toward the skin side along the gluteal cleft, and the part deformed in the convex shape enters in the gluteal cleft, thereby improving fittedness on the gluteal cleft.

Furthermore, the front-surface side concave groove includes the longitudinal direction concave grooves formed in the radial form to be inclined to the outer side in the width direction from the front side toward the back side in the absorbent article. This allows the back side part of the absorbent body to be deformed more flexibly along the curve of the gluteal. That is, with the longitudinal direction concave grooves formed in the radial form, as described above, the separation distance between the adjacent longitudinal direction concave grooves is larger at the position close to the back side than the position close to the crotch side, thereby being able to correspond to the change of the fine curve of the gluteal on the crotch side while being able to corresponding to the large curve of the gluteal on the back side. Therefore, the back side part of the absorbent body can be securely deformed flexibly along the shape of the gluteal.

The invention according to claim 2 is the absorbent article according to claim 1, in which a groove width of the front-surface side concave groove is formed so as to be gradually larger on the back side than the front side in the longitudinal direction of the absorbent article, and is formed so as to be gradually larger on the outer side than the center in the width direction of the absorbent article.

In the above-described invention according to claim 2, to allow the absorbent body to be deformed more easily along the curve of the gluteal, the groove width of the front-surface side concave groove is formed to be larger on the back side than the front side and larger on the outer side than the center side in the width direction. It can be assumed that in the absorbent articles in the above-described Patent Literatures 1 and 2, the section size of the concave groove is almost uniform. Thus when the absorbent article is deformed with the concave groove as an origin, the angle (bent angle) formed by the adjacent small absorption parts is within a certain range where both side walls of the concave groove are in contact in all regions, whereby it is difficult for the absorbent body to be deformed following the degree of the curve of the body varying depending on parts. That is, at a part where the curve of the body is moderate, a gap occurs between both the side walls of the concave groove, and such a part becomes a recessed portion and causes a gap from the skin surface, which allows body liquids to flow easily through the gap. On the other hand, at a part where the curve of the body is sharp, both the side walls of the concave groove are in contact and hardly bent further, so that the bent angle between the adjacent small absorption parts is insufficient. This causes a gap between the front surface of the small absorption part and the skin surface, which allows body liquids to flow easily through the gap. Meanwhile, in the invention, the groove width of the front-surface side concave groove is varied depending on parts, so that the bent angle of the adjacent partitioned absorption parts is also larger on the back side than the front side in the longitudinal direction and larger on the outer side than the center side in the width direction. Therefore, the absorbent body is easily deformed along the shape of the gluteal having a sharper curve on the back side in the longitudinal direction and the outer side in the width direction, thereby improving fittedness on the gluteal.

The invention according to claim 3 is the absorbent article according to claim 1 or 2, in which an embossed groove compressed from the skin side surface toward the non-skin side is formed along a bottom portion of the front-surface side concave groove.

In the above-described invention according to claim 3, the embossed groove compressed from the skin side surface toward the non-skin side is formed along the bottom portion of the front-surface side concave groove. With the embossed groove, the bottom portion of the concave groove is consolidated, and the capillary action of fiber allows body liquids absorbed by the absorbent body to be easily diffused along the concave groove, and prevents leakage of body liquids due to diffusion of the absorbed body liquids in a large range of the absorbent body.

The invention according to claim 4 is the absorbent article according to any one of claims 1 to 3, in which of the longitudinal direction concave groove and the width direction concave groove, one concave groove is formed by an embossed part compressed from the skin side surface of the absorbent body, and the other concave groove is formed by a concave part with a reduced amount of loaded fiber on the skin side of the absorbent body.

In the above-described invention according to claim 4, if the absorbent body is deformed along the shape of the body when attached, it is possible to secure the strength of the absorbent body and prevent a split of the absorbent body because one of the longitudinal direction concave groove and the width direction concave groove is formed by the embossed part.

The invention according to claim 5 is the absorbent article according to any one of claims 1 to 4, in which the gluteal cleft-facing concave groove is formed by an embossed part compressed from the non-skin-side surface toward the skin side, which is formed along the longitudinal direction from the width direction center part in the longitudinal direction middle part of the absorbent body to the back end of the absorbent body.

In the above-described invention according to claim 5, the gluteal cleft-facing concave groove is formed by the embossed part at a given position. This allows the back side part of the absorbent body to be deformed easily along the gluteal cleft, and prevents the absorbent body entered in the gluteal cleft from being split due to the movement of the gluteal and the like.

### Advantageous Effects of Invention

As described above in detail, in the present invention, the back side part of the absorbent article is easily deformed along the shape of a body, thereby improving fittedness.

### Brief Description of Drawings

Fig. 1 is a partially broken development of a sanitary napkin 1 according to the invention.
Fig. 2 is a diagram viewed from the arrow direction of II-II in Fig. 1.
Fig. 3 is a diagram viewed from the arrow direction of III-III in Fig. 1.
Fig. 4 is a plane view of a skin-side surface of an absorbent body 4.
Fig. 5 is a section view illustrating a deformed state of the absorbent body 4 with a front-surface side concave groove 10 as an origin.
Fig. 6 is a section view illustrating a deformed state of the absorbent body 4 with the front-surface side concave groove 10 as an origin according to a modification.
Fig. 7A is a section view illustrating the front-surface side concave groove 10 on the front side in the longitudinal direction or on the center side in the width direction, and Fig. 7B is a section view illustrating the front-surface side concave groove 10 on the back side in the longitudinal direction or on the outer side in the width direction.
Fig. 8 is a section view of the absorbent body 4 in a case where an embossed groove 15 is formed on the bottom portion of the front-surface side concave groove 10. Description of Embodiments

The following will describe an embodiment of the invention with reference to the enclosed drawings.

### [Basic structure of sanitary napkin 1]

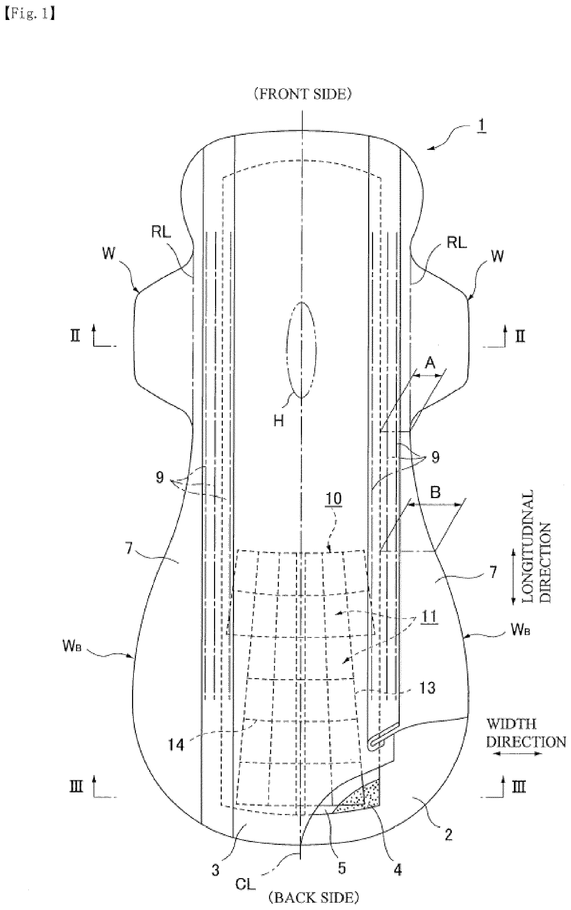

As illustrated in Fig. 1 to Fig. 3, the sanitary napkin 1 of the invention includes a liquid impermeable back-surface sheet 2 formed of a polyethylene sheet or the like, a liquid permeable front-surface sheet 3 allowing rapid permeation of menstrual blood, vaginal discharge, and the like (hereinafter, also referred to collectively as body liquids), an absorbent body 4 made of cotton-like pulp or synthetic pulp interposed between both the sheets 2 and 3, and a side nonwoven fabric 7 provided at both side parts on the skin-contact surface side over the whole length along the longitudinal direction. In addition, in the periphery of the absorbent body 4, the outer edge portions of the liquid impermeable back-surface sheet 2 and the liquid permeable front-surface sheet 3 are jointed at the upper and lower end edge portions of the absorbent body 4 by an adhesive such as hotmelt or joint means such as heat sealing or ultrasonic sealing, and the liquid impermeable back-surface sheet 2 and the side nonwoven fabric 7 extending to the side than the absorbent body 4 are jointed at the both side edge portions of the absorbent body 4 by an adhesive such as hotmelt or joint means such as heat sealing or ultrasonic sealing, whereby a flap part without the interposed absorbent body is formed. In the illustrated example, the absorbent body 4 is surrounded by a wrapping sheet 5 formed of crepe paper, a nonwoven fabric, or the like to keep the shape of the absorbent body 4 and improve diffusibility. However, this wrapping sheet 5 may not be provided. Moreover, a second sheet (not illustrated) formed of a hydrophilic nonwoven fabric or the like with the substantially same shape as the liquid permeable front-surface sheet 3 may be provided adjacent to the non-skin side of the liquid permeable front-surface sheet 3.

Hereinafter, the structure of the sanitary napkin 1 will be further described in more detail. As the liquid impermeable back-surface sheet 2, there is used a sheet material having at least impervious properties such as polyethylene. However, it is preferable to use a material having moisture permeability in the view of preventing stuffiness. As the impervious and moisture permeable sheet material, there is preferably used a microporous sheet obtained by melt kneading an inorganic filler in olefin-based resin such as polyethylene and polypropylene to form a sheet and extending it in a one or two axis direction. The non-skin-side surface (outer surface) of the liquid impermeable back-surface sheet 2 is formed with one or a plurality of strips of adhesive layers (not illustrated) along the longitudinal direction of the napkin, so that the sanitary napkin 1 is fixed to an underwear when attached on a body. The liquid impermeable back-surface sheet 2 may be a poly-laminate nonwoven fabric in which a plastic film and a nonwoven fabric are laminated.

Next, a porous or nonporous nonwoven fabric, a porous plastic sheet, or the like is preferably used as the liquid permeable surface sheet 3. The fiber material forming a nonwoven fabric can be synthetic fiber such as olefin-based fiber of polyethylene, polypropylene, or the like, polyester-based fiber, and polyamide-based fiber, regenerated fiber such as rayon or cupra, and natural fiber such as cotton. There may be used a nonwoven fabric obtained by an appropriate processing method such as the spun lace method, the spun bond method, the thermal bond method, the melt-blown method, or the needle punch method. Among these processing methods, the spun lace method is excellent in flexibility and drape property, and the thermal bond method is excellent in compression restorability for a bulk. In the case where the liquid permeable front-surface sheet 3 has a number of through holes, body fluids are absorbed quickly, thereby achieving excellent dry touch. The fiber of the nonwoven fabric may be either long fiber or short fiber. However, short fiber is preferably used to achieve a towel cloth feeling. Moreover, to facilitate emboss processing, it is preferable to use olefin -based fiber such as polyethylene or polypropylene having a relatively low melting point. Moreover, it is also possible to preferably use conjugated fiber of sheath-core fiber with fiber having a high melting point as a core and fiber having a low melting point as a sheath, side-by-side type fiber, and splittable fiber.

The absorbent body 4 interposed between the liquid impermeable back surface sheet 2 and the liquid permeable surface sheet 3 is made of cotton-like pulp and high absorbent polymer, for example. The absorbent polymer is mixed in the pulp forming the absorbent body, as granular powder, for example. The examples of the pulp include chemical pulp obtained from woods, cellulose fiber such as dissolving pulp, and artificial cellulose fiber such as rayon and acetate. The conifer tree pulp having larger fiber is used more preferably than the broad leaf tree pulp in terms of functions and prices.

Moreover, synthetic fiber may be mixed for the absorbent body 4. As the synthetic fiber, there may be used polyolefin-based fiber such as polyethylene or polypropylene, polyester-based fiber such as polyethylene terephthalate and polybutylene terephthalate, polyamide-based fiber such as nylon, and copolymer of these. The mixture of two kinds of these fiber may be also used. Moreover, it is also possible to use conjugated fiber of sheath-core fiber with fiber having a high melting point as a core and fiber having a low melting point as a sheath, side-by-side type fiber, splittable fiber, and the like. Regarding the synthetic fiber, it is desirable to use synthetic fiber, if it is hydrophobic fiber, on which surface processing is performed by a hydrophilizing agent so as to have affinity relative to body fluids.

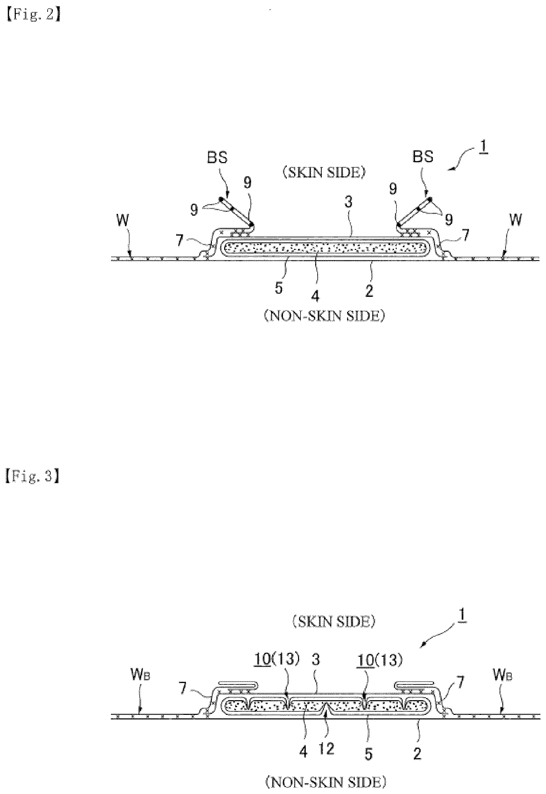

In the illustrated example, the width dimension of the liquid permeable front-surface sheet 3 is slightly larger than the width of the absorbent body 4 only to cover the absorbent body 4, as illustrated in the transverse section views of Fig. 2 and Fig. 3. On the outer side, there is arranged the side nonwoven fabric 7 separate from the liquid permeable front-surface sheet 3, concretely, the side nonwoven fabric 7 made of a nonwoven fabric material subjected to appropriate water repellent treatment or hydrophilic treatment in accordance with the object such as prevention of permeation of menstrual blood, vaginal discharge, and the like or improvement of skin touch feeling. It is possible to use the side nonwoven fabric 7 formed by processing the material such as natural fiber, synthetic fiber, or regenerated fiber by an appropriate processing method. It is preferable to use a nonwoven fabric having air permeability with a less base weight so as to remove stiffness and prevent stuffiness. To be more specific, it is preferable to use a nonwoven fabric with a base weight of 13 to 23 g/m², and to securely prevent permeation of body liquids, it is preferable to use a water repellent nonwoven fabric coated with a silicone-based, paraffin-based, or alkyl chromic chloride-based water repellent agent, or the like.

As illustrated in Fig. 2 and Fig. 3, in the side nonwoven fabric 7, the outer side part than the middle part in the width direction is adhered by an adhesive such as hotmelt in ae range from a given inner side position to the outer edge of the liquid impermeable back-surface sheet 2, and a layered sheet part of the side nonwoven fabric 7 and the liquid impermeable back-surface sheet 2 forms a flap part without the interposed absorbent body 4 at both side parts of the absorbent body 4. The flap part may include a pair of right and left wing-shaped flaps W, W formed at absorbent body side part positions substantially corresponding to a body liquid discharging area H, and hip holding flaps W_{B}, W_{B} formed at gluteal side (back part side) positions than the wing-shaped flaps W, W. Each of these wing-shaped flaps W, W and hip holding flaps W_{B}, W_{B} has an adhesive layer (not illustrated) on the outer surface side thereof. When attached on shorts the wing-shaped flaps W, W are folded to the opposite side at folding line RL positions of the base end part, wound around a crotch part of the shorts, and fixed thereto, while the hip holding flaps W_{B} are fixed to the inner surface of the shorts.

Meanwhile, the inner side part of the nonwoven fabric 7 is folded to be almost double, and there are provided, in the double sheet, one or a plurality of (three in the illustrated example) filamentous elastic stretching members 9, 9, ... so that the both ends or appropriate positions in the longitudinal direction are fixed in the middle part in the height direction in the state where the both ends or the appropriate positions in the longitudinal direction are fixed. The double sheet part is adhered to the absorbent body 4 side in the state where the front and back end portions thereof are folded outward once and layered, as illustrated in Fig. 3. Thus, as illustrated in Fig. 2, there are formed symmetrically linear three-dimensional gathers BS, BS raised to the front-surface side while inclined outward.

### [Concave groove]

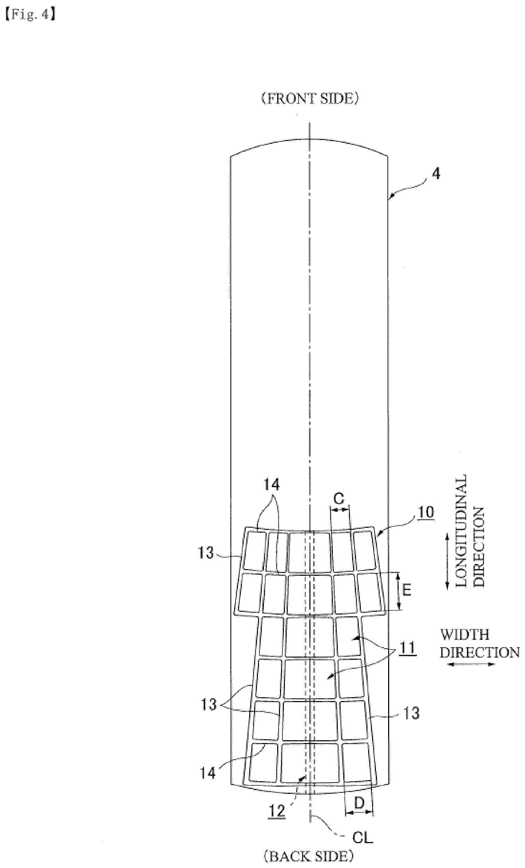

The following will describe a concave groove formed on the absorbent body 4. As illustrated in Fig. 4, on the skin-side surface of the back side part of the absorbent body 4, there are formed a front-surface side concave groove 10 recessed to the non-skin side in a given pattern, and a plurality of partitioned absorption parts 11, 11, ... partitioned by the front-surface side concave groove 10. In addition, at a position facing the gluteal cleft of a wearer on the non-skin-side surface of the back side part of the absorbent body 4, there is formed a gluteal cleft-facing concave groove 12 recessed to the skin side along the gluteal cleft.

As illustrated in Fig. 1, the back side part of the absorbent body 4 where the front-surface side concave groove 10, the partitioned absorption part 11, and the gluteal cleft-facing concave groove 12 are formed is an area on the back side in the longitudinal direction than the wing-shaped flap W, that is, and area on the back side in the longitudinal direction than the area corresponding to a body liquid discharging area H of the wearer. More preferably, the back side part of the absorbent body 4 is an area where hip holding flaps W_{B} are formed, on the back side in the longitudinal direction than a region at which the hip holding flap W_{B} gradually projecting to the outer side in the width direction toward the back side has a given width or more, concretely, on the back side in the longitudinal direction than a position at which a ratio B/A between a flap width A at a part where the length from the side edge of the absorbent body 4 to the side edge of the sanitary napkin 1 at the back end portion of the wing-shaped flap W is smallest and a flap width B at the front ends of the front-surface side concave groove 10 and the gluteal cleft-facing concave groove 12 is about 2.0 to 3.0.

The front-surface side concave groove 10, the partitioned absorption part 11, and the gluteal cleft-facing concave groove 12 are formed only on the back side part of the sanitary napkin 1, and are not formed on the area on the front side than such a back side part. The front-surface side concave groove 10, the partitioned absorption part 11, and the gluteal cleft-facing concave groove 12 allow the absorbent body to be deformed easily along the shape of the gluteal. The deformation state of the absorbent body is different on the front side part, and thus it is rather preferable not to provide them on the front side part.

The skin-side surface of the absorbent body 4 where the front-surface side concave groove 10 is formed is a skin-contact surface of the absorbent body 4, while the non-skin-side surface where the gluteal cleft-facing concave groove 12 is formed is a skin-noncontact surface (underwear side surface) of the absorbent body 4.

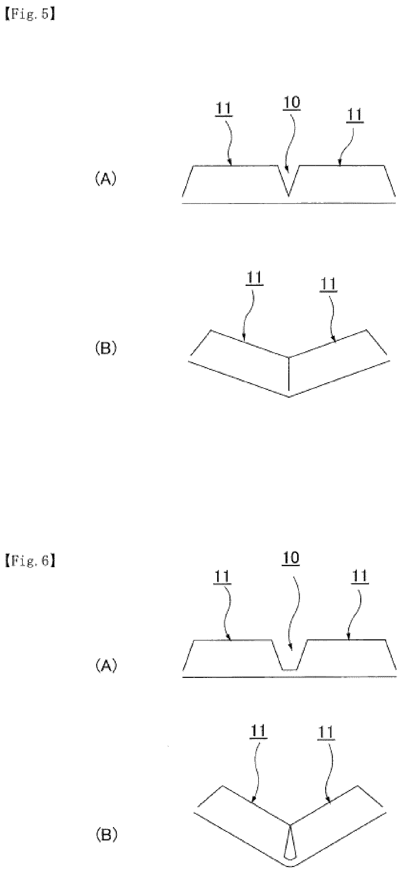

In the sanitary napkin 1, the skin-side surface of the back side part of the absorbent body 4 has the front-surface side concave groove 10 recessed to the non-skin side in a given pattern and a plurality of partitioned absorption parts 11 partitioned by the front-surface side concave groove 10. Thus, as illustrated in Fig. 5 and Fig. 6, a part between the adjacent partitioned absorption parts 11, 11 is bent inward, with the front-surface side concave groove 10 recessed to the non-skin side as an origin, so as to close both side walls of the front-surface side concave groove 10. This allows the back side part of the absorbent body to be bulged outward as a whole and deformed flexibly. Therefore, the back side part of the absorbent body 4 is deformed flexibly along the curve of the gluteal, thus improving fittedness on the gluteal.

Moreover, the non-skin-side surface of the back side part of the absorbent body 4 has the gluteal cleft-facing concave groove 12 at a position corresponding to the gluteal cleft. Thus, with the gluteal cleft-facing concave groove 12 as a flexible axis, the absorbent body 4 is easily deformed in a convex shape toward the skin side along the gluteal cleft, and the part deformed in the convex shape enters in the gluteal cleft, thereby improving fittedness on the gluteal cleft.

The front-surface side concave groove 10 is a concave groove recessed to the non-skin side, which is formed on the skin-side surface of the back side part of the absorbent body 4. The front-surface side concave groove 10 may be formed so that only the absorbent body 4 is recessed to the non-skin side, formed so that the wrapping sheet 5 and the absorbent body 4 are integrally recessed to the non-skin side, or formed so that the liquid permeable front-surface sheet 3 to the absorbent body 4 are integrally recessed to the non-skin side. In either case, it is only needed that the absorbent body 4 is easily bent with the front-surface side concave groove 10 as an origin.

The front-surface side concave groove 10 is a part where the skin-side surface is recessed to the non-skin side relative to the peripheral parts to have a small thickness, and is a part where the bending rigidity of the absorbent body 4 is smaller. The absorbent body 4 is not divided by the front-surface side concave groove 10, and the absorbent body exists on the bottom portion of the front-surface side concave groove 10.

As illustrated in Fig. 4, the front-surface side concave groove 10 includes a plurality of longitudinal direction concave grooves 13, 13 ...... arranged along the substantially longitudinal direction of the sanitary napkin 1 with intervals in the width direction, and a plurality of width direction concave grooves 14, 14 ...... arranged along the substantially width direction of the sanitary napkin 1 with intervals in the longitudinal direction.

The longitudinal direction concave grooves are formed from the middle position toward the back side in the longitudinal direction to a position in the vicinity of the back side end edge or the side end edge of the absorbent body 4, and the adjacent longitudinal direction concave grooves 13, 13 are partitioned by the width direction concave grooves 14 with given intervals in the longitudinal direction.

The longitudinal direction concave groove 13 is formed linearly, and the width direction concave groove 14 connecting the adjacent longitudinal direction concave grooves 13, 13 is formed linearly to be substantially orthogonal to the longitudinal direction concave groove 13. The width direction concave groove 14 is continuous from the end edge of the width direction concave groove 14 arranged between a pair of longitudinal direction concave grooves 13, 13, and extends toward the next adjacent longitudinal direction concave groove 13. Thus, the width direction concave grooves 14 as a whole are formed linearly to be continuous in the width direction of the absorbent body 4.

The longitudinal direction concave grooves 13 are formed in a radial form to be inclined outward in the width direction from the front side toward the back side in the sanitary napkin 1. That is, a separation distance between the adjacent longitudinal direction concave grooves 13, 13 is formed relatively larger on the back side than the front side in the sanitary napkin 1. The radial form is configured by a plurality of straight lines having different angles with a longitudinal direction center line CL toward the outer side in the width direction on the back side in the longitudinal direction, with one point or a plurality of points adjacent to one other on the longitudinal direction center line CL as a center.

In the sanitary napkin 1, the longitudinal direction concave grooves 13 are formed in the radial form to be inclined to the outer side in the width direction from the front side to the back side in the sanitary napkin 1. This allows the back side part of the absorbent body 4 to be deformed more flexibly along the curve of the gluteal. That is, with the longitudinal direction concave grooves 13 formed in the radial form, the separation distance between the adjacent longitudinal direction concave grooves 13, 13 is larger at the position close to the back side than the position close to the crotch side, thereby corresponding to the change of the fine curve of the gluteal on the crotch side while corresponding to the large curve of the gluteal on the back side. Therefore, the back side part of the absorbent body 4 can be securely deformed flexibly along the shape of the gluteal.

The thickness of the absorbent body 4 where the front-surface side concave groove 10, the partitioned absorption part 11, and the gluteal cleft-facing concave groove 12 are formed is 3 to 20 mm, and is preferably 5 to 15 mm.

The separation distance between the adjacent longitudinal direction concave grooves 13, 13 (length in the width direction of the partitioned absorption part 11) is 5 to 30 mm, and is preferably 10 to 20 mm. As illustrated in Fig. 4, the separation distance between the adjacent longitudinal direction concave groove 13, 13 is set so that the ratio D/C between a separation distance C at the front-side end portion and a separation distance D at the back-side end portion is 1.3 to 4.0 time, and is preferably 1.5 to 2.5 times. The ratio D/C of the separation distance between the front-side end portion and the back-side end portion is a ratio in the case where the longitudinal direction concave groove 13 is formed to the vicinity of the back side end edge of the absorbent body 4. As in the illustrated example, in the case where the longitudinal direction concave groove does not extend to the back side end edge of the absorbent body 4, such as the longitudinal direction concave groove 13 outermost in the width direction, it is possible, similarly to the longitudinal direction concave groove 13 formed to the vicinity of the back side end edge of the absorbent body 4 on the inner side in the width direction, to obtain the separation distance C at the front side end portion and the separation distance D at the back side end portion D for the case where the longitudinal direction concave groove 13 is extended to the vicinity of the back side end edge of the absorbent body 4.

The width direction concave groove 14 is preferably formed linearly to be orthogonal to a virtual line (not illustrated) passing the middle part between the adjacent longitudinal direction concave grooves 13, 13.

The longitudinal direction concave grooves 13 are formed in the radial form, and the width direction concave grooves 14 are formed to be substantially orthogonal to the virtual line passing the middle part between the adjacent longitudinal direction concave grooves 13, 13. In this manner, the width direction concave grooves 14 are formed in a polygonal line shapes refracting in the longitudinal direction concave grooves 13 in the width direction of the absorbent body 4.

A separation distance E between the width direction concave grooves 14 adjacent to each other in the longitudinal direction (longitudinal direction length of the partitioned absorption part 11) may be formed to be approximately uniform over the whole length, or may be different depending on a part so as to be adjusted to the curve of the gluteal. If the distance is made different, it is preferable that the size on the front side in the longitudinal direction is relatively small, and the size on the back side in the longitudinal direction is relatively large. The separation distance E is 10 to 50 mm, and is preferably 15 to 30 mm.

The front-surface side concave groove 10 is preferably formed in the middle part of the absorbent body 4 without reaching the back-side end edge or the side end edge of the absorbent body 4. That is, it is preferable to arrange, between the end edge of the front-surface side concave groove 10 and the end edge of the absorbent body 4, a margin part where the skin-side surface of the absorbent body is not recessed to the non-skin side. In this manner, it is possible to prevent a split of the absorbent body 4 if the absorbent body 4 is deformed when attached.

The longitudinal direction concave grooves 13 are arranged symmetrically to the longitudinal direction center line CL of the absorbent body 4, as illustrated in Fig. 4. It is preferable that the longitudinal direction concave grooves 13 are provided on each of the left and right sides of the longitudinal direction center line CL of the absorbent body 4 and are not provided on the longitudinal direction center line CL. On the longitudinal direction center line, the gluteal cleft-facing concave groove 12 recessed to the skin side is provided on the non-skin-side surface. Thus, it is not preferable to provide a concave groove recessed from the skin-side surface to the non-skin side.

The section shape of the front-surface side concave groove 10 may be triangular (Fig. 5), trapezoidal (Fig. 6), U-shaped, or the like and is not limited particularly. However, as illustrated in Fig. 5, it is preferable to form the front-surface side concave groove 10 to be substantially triangular so that both side walls cross or adjacent to each other on the bottom portion. In this manner, if the absorbent body 4 is deformed when attached, with the front-surface side concave groove 10 as an origin, both side walls are brought into contact substantially without any gap so as to close the front-surface side concave groove 10, as illustrated in Fig. 5B. Thus, the flow of body liquids through the gap hardly occurs. Meanwhile, in the case where the section shape of the front-surface side concave groove 10 is trapezoidal, as illustrated in Fig. 6, when if absorbent body 4 is deformed when attached, with the front-surface side concave groove 10 as an origin, the groove top portions of both side walls are brought into contact, while the groove bottom portions are deformed to be separate from each other, as illustrated in Fig. 6B.

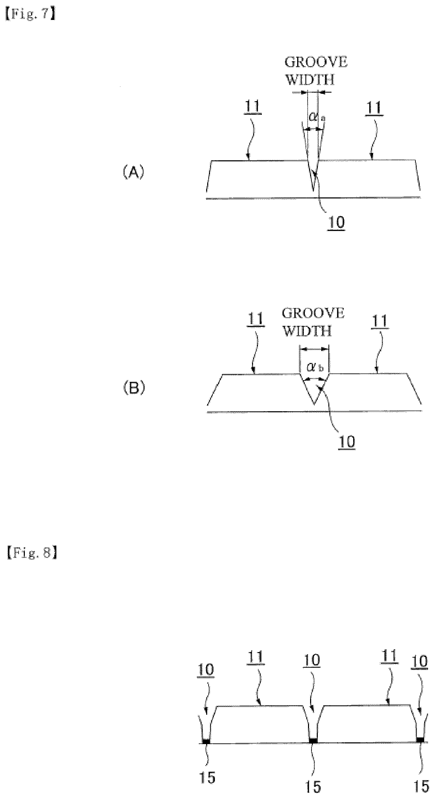

The groove width of the front-surface side concave groove 10 may be formed to be approximately uniform over the whole groove. However, as illustrated in Fig. 4 and Fig. 7, it is preferable to form the front-surface side concave groove 10 so as to be gradually larger in width from the front side toward the back side in the longitudinal direction of the sanitary napkin 1, and so as to be gradually larger in width from the center toward the outer side in the width direction of the sanitary napkin 1. This allows the absorbent body 4 to be deformed still more easily along the curve of the gluteal. That is, the bend angle of the adjacent partitioned absorption parts 11, 11 is large at a part where the groove width of the front-surface side concave groove 10 is large, while the bend angle of the adjacent partitioned absorption parts 11, 11 is small at a part where the groove width of the front-surface side concave groove 10 is small. Therefore, the absorbent body 4 is easily deformed along the shape of the gluteal having a sharper curve on the back side in the longitudinal direction and on the outer side in the width direction. Fig. 7A illustrates the front-surface side concave groove 10 with a relatively small groove width, and Fig. 7B illustrates the front-surface side concave groove 10 with a relatively large groove width. Here, the groove width indicates a separation distance between both side walls at the groove top portion.

In the case where the section shape of the front-surface side concave groove 10 is substantially triangular, the angle αₐ formed by both side walls is relatively small if the groove width is made relatively small on the front side and the center side in the width direction of the front-surface side concave groove 10, while the angle α_{b} formed by both side walls is relatively large if the groove width is made relatively large on the back side, and the outer side in the width direction of the front-surface side concave groove 10. The αₐ is 10 to 45°, and is preferably 20 to 30°, and the α_{b} is 30 to 90°, and is preferably 40 to 50°.

The groove depth of the front-surface side concave groove 10 is preferably 20 to 50% relative to the thickness of the absorbent body 4.

It is preferable that the front-surface side concave groove 10 has an embossed groove 15 compressed from the skin-side surface to the non-skin side along the groove bottom portion, as illustrated in Fig. 8. With the embossed groove 15, the bottom portion of the front-surface side concave groove 10 is consolidated, and the capillary action of fiber allows body liquids absorbed by the absorbent body 4 to be easily diffused along the front-surface side concave groove 10, and prevents leakage of body liquids due to diffusion of the absorbed body liquids in a large range of the absorbent body 4.

The groove width of the embossed groove 15 is preferably smaller than the groove width of the front-surface side concave groove 10 (separation distance between both side walls at the groove top portion). The embossed groove 15 may be provided in the whole of the front-surface side concave groove 10 or in a part thereof. In the case where the embossed groove 15 is provided in a part, the embossed groove 15 may be provided only in the longitudinal direction concave groove 13 and may not be provided in the width direction concave groove 14 so as to accelerate diffusion of body liquids to the longitudinal direction of the absorbent body 4. Alternatively, the embossed groove 15 may be provided only in the front-surface side concave groove 10 on the center side in the width direction and on the center side in the longitudinal direction of the absorbent body 4, and may not be provided on the front-surface side concave groove 10 around the end edge of the absorbent body 4 to accelerate diffusion of body liquids at the middle part of the absorbent body 4 and prevent diffusion at the end edge portion of the absorbent body 4.

To form the front-surface side concave groove 10, it is possible to use known aspects for forming a concave groove on an absorbent body without any restriction. For example, there may be adopted an aspect of reducing the amount of loaded fiber on the skin side of the absorbent body 4 or an aspect of compressing the absorbent body 4 from the skin-side surface of the absorbent body 4.

As a preferable embodiment, the longitudinal direction concave groove 13 may be configured by an embossed part compressed from the skin-side surface of the absorbent body 4, and the width direction concave groove 14 may be formed by a concave part with a reduced amount of loaded fiber on the skin side of the absorbent body 4. Therefore, if the absorbent body 4 is deformed along the shape of the body when attached, it is possible, with the longitudinal direction concave groove 13 formed by the embossed part, to secure the strength of the absorbent body 4 and prevent a split of the absorbent body 4. The "embossed part compressed from the skin-side surface of the absorbent body 4" may be an embossed part formed by compressing the absorbent body 4 (or the wrapping sheet 5) so that only the absorbent body 4 (or only the wrapping sheet 5 and the absorbent body 4) is recessed, or an embossed part formed by compressing the liquid permeable front-surface sheet 3 so that the liquid permeable front-surface sheet 3 to the absorbent body 4 are recessed integrally.

The partitioned absorption part 11 is a square shaped absorbent body part whose periphery is partitioned by the front-surface side concave groove 10, and is formed to be relatively thicker than the front-surface side concave groove 10. To be more specific, each of the both sides is partitioned by the longitudinal direction concave groove 13, and each of the front and back sides is partitioned by the width direction concave groove 14. A plurality of partitioned absorption parts 11 are formed along the longitudinal direction and the width direction of the absorbent body 4. The size of the partitioned absorption parts 11 is not uniform. With the longitudinal direction concave groove 13 formed in the radial form, as described above, the partitioned absorption part 11 is formed to have a relatively larger area toward the back side of the absorbent body 4.

The partitioned absorption part 11 crossing the longitudinal direction center line CL is preferably formed to have a relatively larger area than the partitioned absorption parts 11 formed in other regions. The partitioned absorption part 11 crossing the longitudinal direction center line CL is a part that is bent in the width direction by the gluteal cleft-facing concave groove 12 provided on the non-skin-side surface of the absorbent body 4 and enters in the gluteal cleft when attached. Thus, if such a partitioned absorption part 11 is formed to have an excessively small area, it is hardly bent, thereby causing a split of the absorbent body 4 entered in the gluteal cleft more easily. The area of the partitioned absorption part 11 crossing the longitudinal direction center line CL is preferably 1.5 to 3 times the area of other regions, and is most preferably about twice.

The adjacent partitioned absorption parts 11, 11 are arranged in parallel through the front-surface side concave groove 10 in the state where the sanitary napkin 1 before attached is developed, as illustrated in Fig. 5A and Fig. 6A. In the state where the sanitary napkin 1 is deformed with the front-surface side concave groove 10 as an origin when attached, as illustrated in Fig. 5B and Fig. 6B, the adjacent partitioned absorption parts 11, 11 are arranged almost adjacent to each other with a given bent angle. The partitioned absorption part 11 is not bent when attached, and the skin-side surface of the partitioned absorption part 11 faces the skin surface.

The gluteal cleft-facing concave groove 12 is a concave groove recessed to the skin side, which is formed on the non-skin-side surface of the back side part of the absorbent body 4. In the gluteal cleft-facing concave groove 12, only the absorbent body 4 may be recessed to the non-skin side, or the wrapping sheet 5 and the absorbent body 4 may be recessed integrally to the non-skin side.

The gluteal cleft-facing concave groove 12 is formed in one strip linear shape along the gluteal cleft at a position corresponding to the gluteal cleft on the non-skin-side surface of the back side part of the absorbent body 4. That is, it is preferable to form the gluteal cleft-facing concave groove 12 along the longitudinal direction from the center part in the width direction of the middle part in the longitudinal direction of the absorbent body 4 to the back end of the absorbent body 4. In this manner, with the gluteal cleft-facing concave groove 12 as a flexible axis, the center part in the width direction of the absorbent body 4 easily projects to the skin side along the longitudinal direction, and a part deformed in the convex form enters in the gluteal cleft, thereby improving fittedness on the body.

The front side end edge of the gluteal cleft-facing concave groove 12 is preferably arranged to substantially match the front side end edge of the front-surface side concave groove 10. In this manner, the deformation along the curve of the gluteal by the front-surface side concave groove 10 and the deformation along the gluteal cleft by the gluteal cleft-facing concave groove 12 occur from the substantially matched position. Meanwhile, the back side end edge of the gluteal cleft-facing concave groove 12 is preferably formed to the back side end edge of the absorbent body 4. That is, the gluteal cleft-facing concave groove 12 is formed to project to the back side than the back side end edge of the front-surface side concave groove 10. In this manner, the back side part including the back side end edge of the absorbent body 4 is easily deformed along the gluteal cleft, thereby securely improving fittedness on the gluteal cleft.

The gluteal cleft-facing concave groove 12 is preferably formed by an embossed part compressed from the non-skin-side surface to the skin side of the absorbent body 4. This prevents the part entered in the gluteal cleft from being split due to the movement of the gluteal cleft.

Similarly to the above-described front-surface side concave groove 10, the section shape of the gluteal cleft-facing concave groove 12 may be also triangular (Fig. 5), trapezoidal (Fig. 6), U-shaped, or the like, and is not limited particularly.

The groove width of the gluteal cleft-facing concave groove 12 is formed to be relatively larger than the front-surface side concave groove 10. To be more specific, it is preferably 2 to 20 mm. The gluteal cleft-facing concave groove 12 is preferably formed with a uniform groove width over the whole length.

The groove depth of the gluteal cleft-facing concave groove 12 is preferably 20 to 50% relative to the thickness of the absorbent body 4.

### [Other embodiments]

(1) In the sanitary napkin 1 illustrated in Fig. 1, a concave groove is not formed at the front side part than the front-surface side concave groove 10. However, a concave groove recessed to the non-skin side may be formed in an appropriate pattern on the skin-side surface of the absorbent body 4.
(2) A middle-height portion (not illustrated) of the absorbent body, which is made thicker to the skin side, may be provided in the area including a region corresponding to the body liquid discharging area H of the absorbent body 4. The middle-height portion is adjacent to the skin side surface of the absorbent body 4, arranged in the center part in the width direction of the absorbent body 4, and formed with a smaller width size and longitudinal size than the absorbent body 4.

### Reference Signs List

- 1: sanitary napkin
- 2: liquid impermeable back-surface sheet
- 3: liquid permeable front-surface sheet
- 4: absorbent body
- 5: wrapping sheet
- 7: side nonwoven fabric
- 10: front-surface side concave groove
- 11: partitioned absorption part
- 12: gluteal cleft-facing concave groove
- 13: longitudinal direction concave groove
- 14: width direction concave groove
- 15: embossed groove

## Claims

1. An absorbent article (1), comprising at least:
an absorbent body (4), wherein
a skin side surface of a back side part of the absorbent body (4) includes a front-surface side concave groove (10) recessed to a non-skin side formed in a given pattern and a plurality of partitioned absorption parts (11) partitioned by the front-surface side concave groove (10), and a non-skin-side surface of the back side part of the absorbent body (4) includes a gluteal cleft-facing concave groove (12) recessed to a skin side at a position corresponding to a gluteal cleft, the front-surface side concave groove (10) includes a plurality of longitudinal direction concave grooves (13) arranged along a substantially longitudinal direction of the absorbent article (1) with intervals in a width direction and a plurality of width direction concave grooves (14) arranged along the substantially width direction of the absorbent article (1) with intervals in the longitudinal direction, and the longitudinal direction concave grooves (13) are formed in a radial form inclined outward in the width direction from a front side toward a back side of the absorbent article (1).

2. The absorbent article (1) according to claim 1, wherein a groove width of the front-surface side concave groove (10) is formed so as to be gradually larger on the back side than the front side in the longitudinal direction of the absorbent article (1), and is formed so as to be gradually larger on the outer side than the center in the width direction of the absorbent article (1).

3. The absorbent article (1) according to claim 1 or 2, wherein an embossed groove (15) compressed from the skin side surface toward the non-skin side is formed along a bottom portion of the front-surface side concave groove (10).

4. The absorbent article (1) according to any one of claims 1 to 3, wherein of the longitudinal direction concave groove (13) and the width direction concave groove (14), one concave groove is formed by an embossed part compressed from the skin side surface of the absorbent body (4), and the other concave groove is formed by a concave part with a reduced amount of loaded fiber on the skin side of the absorbent body (4),

5. The absorbent article (1) according to any one of claims 1 to 4, wherein the gluteal cleft-facing concave groove (12) is formed by an embossed part compressed from the non-skin-side surface toward the skin side, which is formed along the longitudinal direction from a width direction center part in a longitudinal direction middle part of the absorbent body (4) to a back end of the absorbent body (4).

## Patentansprüche

1. Absorbierender Artikel, umfassend mindestens:
einen absorbierenden Körper (4), wobei
eine hautseitige Oberfläche eines rückseitigen Abschnitts des absorbierenden Körpers (4) eine in Richtung der hautabgewandten Seite zurückgesetzte, vorderflächenseitige konkave Rille (10), die in einem vorgegebenen Muster ausgebildet ist, und eine Vielzahl von abgeteilten Absorptionsabschnitten (11), die durch die vorderflächenseitige konkave Rille (10) abgeteilt sind, umfasst, und eine nicht-hautseitige Oberfläche des rückseitigen Abschnitts des absorbierenden Körpers (4) eine der Gesäßspalte zugewandte konkave Rille (12), die an einer der Gesäßspalte entsprechenden Position in Richtung der Hautseite zurückgesetzt ist, umfasst, die vorderflächenseitige konkave Rille (10) eine Vielzahl von in Längsrichtung verlaufenden konkaven Rillen (13), die im Wesentlichen entlang der Längsrichtung des absorbierenden Artikels (1) in Intervallen in der Breitenrichtung angeordnet sind, und eine Vielzahl von in Breitenrichtung verlaufenden konkaven Rillen (14), die im Wesentlichen entlang der Breitenrichtung des absorbierenden Artikels (1) in Intervallen in der Längsrichtung angeordnet sind, umfasst, und die in Längsrichtung verlaufenden konkaven Rillen (13) in einer radialen Form nach außen in Breitenrichtung geneigt von einer Vorderseite zu einer Hinterseite des absorbierenden Artikels (1) ausgebildet sind.

2. Absorbierender Artikel (1) gemäß Anspruch 1, wobei eine Rillenbreite der vorderflächenseitigen konkaven Rille (10) so ausgebildet ist, dass sie auf der Rückseite graduell größer als auf der Vorderseite in der Längsrichtung des absorbierenden Artikels (1) ist, und so ausgebildet ist, dass sie an der äußeren Seite graduell größer als in der Mitte in der Breitenrichtung des absorbierenden Artikels (1) ist.

3. Absorbierender Artikel (1) gemäß Anspruch 1 oder Anspruch 2, wobei eine geprägte Rille (15), die von der hautseitigen Oberfläche in Richtung der hautabgewandten Seite hin komprimiert ist, entlang eines Bodenabschnitts der vorderflächenseitigen konkaven Rille (10) ausgebildet ist.

4. Absorbierender Artikel (1) gemäß einem der Ansprüche 1 bis 3, wobei die eine konkave Rille von der konkaven Rille in Längsrichtung (13) und der konkaven Rille in Breitenrichtung (14) durch einen geprägten Abschnitt gebildet wird, der von der hautseitigen Oberfläche des absorbierenden Körpers her (4) komprimiert ist, und die andere konkave Rille durch einen konkaven Abschnitt mit einer verringerten Menge geladener Fasern auf der Hautseite des absorbierenden Körpers (4) ausgebildet wird.

5. Absorbierender Artikel (1) gemäß einem der Ansprüche 1 bis 4, wobei die der Gesäßspalte zugewandte konkave Rille (12) durch einen geprägten Teil, der von der nichthautseitigen Oberfläche zu der Hautseite hin komprimiert ist, gebildet wird, der entlang der Längsrichtung von einem Mittelteil in Breitenrichtung in einem Mittelteil in Längsrichtung des absorbierenden Körpers (4) zu einem hinteren Ende des absorbierenden Körpers (4) ausgebildet ist.

## Revendications

1. Article absorbant (1) comprenant au moins :
un corps absorbant (4), dans lequel
une surface côté peau d'une partie côté arrière du corps absorbant (4) comprend une rainure concave côté surface avant (10) en retrait vers un côté opposé à la peau formée en un motif donné et une pluralité de parties d'absorption cloisonnées (11) cloisonnées par la rainure concave côté surface avant (10), et une surface opposée à la peau de la partie côté arrière du corps absorbant (4) comprend une rainure concave face au pli inter-fessier (12) en retrait vers un côté peau au niveau d'une position correspondant au pli inter-fessier, la rainure concave côté surface avant (10) comprend une pluralité de rainures concaves longitudinales (13) agencées le long d'une direction sensiblement longitudinale de l'article absorbant (1) avec des intervalles dans une direction transversale et une pluralité de rainures concaves transversales (14) agencées le long de la direction sensiblement transversale de l'article absorbant (1) avec des intervalles dans la direction longitudinale, et les rainures concaves longitudinales (13) sont formées en une forme radiale inclinée vers l'extérieur dans la direction transversale depuis un côté avant vers un côté arrière de l'article absorbant (1).

2. Article absorbant (1) selon la revendication 1, dans lequel une largeur de rainure de la rainure concave côté surface avant (10) est formée pour être progressivement plus grande sur le côté arrière que sur le côté avant dans la direction longitudinale de l'article absorbant (1), et est formée pour être progressivement plus grande sur le côté extérieur qu'au centre dans la direction transversale de l'article absorbant (1).

3. Article absorbant (1) selon la revendication 1 ou 2, dans lequel une rainure bosselée (15) compressée depuis la surface côté peau vers la surface opposée à la peau est formée le long d'une partie inférieure de la rainure concave côté surface avant (10).

4. Article absorbant (1) selon l'une quelconque des revendications 1 à 3, dans lequel de la rainure concave longitudinale (13) et de la rainure concave transversale (14), une rainure concave est formée par une partie bosselée comprimée depuis la surface côté peau du corps absorbant (4), et l'autre rainure concave est formée par une partie concave avec une quantité réduite de fibres chargées sur le côté peau du corps absorbant (4).

5. Article absorbant (1) selon l'une quelconque des revendications 1 à 4, dans lequel la rainure concave face au pli inter-fessier (12) est formée par une partie bosselée comprimée depuis la surface opposée à la peau en direction du côté peau, qui est formée le long de la direction longitudinale depuis une partie centrale transversale dans une partie intermédiaire longitudinale du corps absorbant (4) vers une extrémité arrière du corps absorbant (4).
